(19) Europäisches Patentamt
European Patent Office
Office européen des brevets

(11) Publication number : 0 331 382 B1

(12) EUROPEAN PATENT SPECIFICATION

(45) Date of publication of patent specification : 09.09.92 Bulletin 92/37

(51) Int. Cl.$^5$ : A61K 47/00

(21) Application number : 89301896.0

(22) Date of filing : 24.02.89

# (54) Transdermal flux enhancing compositions.

(30) Priority : 29.02.88 US 161926

(43) Date of publication of application : 06.09.89 Bulletin 89/36

(45) Publication of the grant of the patent : 09.09.92 Bulletin 92/37

(84) Designated Contracting States : AT BE CH DE ES FR GB GR IT LI LU NL SE

(56) References cited :
EP-A- 0 129 284
EP-A- 0 271 983
JOURNAL OF PHARMACY AND PHARMACOLOGY, vol. 37, no. 10, October 1985, pages 725-727, Pharm. Soc. of Great Britain, London, GBJ. HADGRAFT et al.: "Facilitated transport of sodium salicylate across an artificial lipid membrane by Azone"
JOURNAL OF PHARMACEUTICL SCIENCES, vol. 73, no. 8, August 1984, pages 1153-1156, Am. Pharm. Ass., US E.R. COOPER: "Increasedskin permeability for lipophilic molecules"

(56) References cited :
DRUG DEVELOPMENT AND INDUSTRIAL PHARMACY, vol. 9, no. 4, 1983, pages 725-744, Marcel Dekker, Inc., US R.B. STOUGHTON et al.:"Azone: A new non-toxic enhancer of cutaneous penetration"
ARCHIVES OF DERMATOLOGY; vol. 118, July 1982, pages 474-477, Am. Med. Ass., Chicago, US R.B. STOUGHTON: "Enhancedpercutaneous penetratin with 1-do-decylazacycloheptan-2-one"

(73) Proprietor : PFIZER INC.
235 East 42nd Street
New York, N.Y. 10017 (US)

(72) Inventor : Potts, Russell Owen
73-A Fenwood Drive
Old Saybrook Connecticut (US)
Inventor : Francoeur, Michael Lee
6, Webster Road
East Lyme Connecticut (US)

(74) Representative : Bradbrook, Geoffrey William et al
PFIZER LIMITED Ramsgate Road
Sandwich Kent, CT13 9NJ (GB)

EP 0 331 382 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to flux enhancing pharmaceutical compositions for transdermal administration to a human or lower animal subject.

The following patents to Rajadhyaksha issued from 1976 to 1984 disclose methods and compositions employing 1-alkylazacycloheptan-2-ones and homologs thereof for enhanced penetration of pharmacologically active agents through human and animal skin;

U.S. 3,989,816; U.S. 4,316,893; U.S. 4,405,616 and 4,444,762.

Stoughton, Arch. Derm., 118, 474-477 (1982) relates to 1-dodecylazacycloheptan-2-one, referred to herein as Azone, and its ability to enhance percutaneous penetration.

Cooper, U.S. 4,557,934 and 4,537,776, discloses topical compositions of nonsteroidal antiinflammatory compounds, antiviral agents, antitussives and other drugs containing ethanol, certain glycols, pyrrolidone, 1-(2-hydroxyethyl)-aza-cyclopentan-2-one and from 1-35% 1-dodecylazacycloheptan-2-one (Azone).

Cooper, J. Pharm. Sci., 73, 1153-1156 (1984) discloses a method for increased transport of nonpolar molecules like salicylic acid through skin by adding fatty alcohols or fatty acids to transdermal formulations in various glycol solvents.

EP-A-0129284 describes topical pharmaceutical compositions containing 1-dodecyl-azacycloheptan-2-one or oleic acid, including compositions containing hydrocortisone or triamcinolone and 25% by weight of propylene glycol, 30% by weight of ethanol, and water.

Akhter and Barry, J. Pharm. Pharmacol., 36, 7P (1984), report that oleic acid and Azone enhance dermal penetration of flurbiprofen formulations in propylene glycol and other solvents.

EP43738 discloses a binary dermal penetration enhancing vehicle for antiinflammatory agents containing a $C_3$-$C_4$-diol, diol ester or diol ether and a cell envelope-disordering compound selected from, inter alia, the lower alkyl esters of $C_{12}$-$C_{14}$ fatty acids, oleic acid, lauryl acetate and myristyl acetate.

Patel, et al., Journ. Soc. Cosmetic Chem. 36, 303-311 (1985) has noted that propylene glycol, a common constituent of prior art pharmaceutical formulations for transdermal use, causes irritation and/or sensitization when its concentration exceeds ten percent.

U.S. 4,572,909, issued February 25, 1986 discloses amlodipine, 2-[(2-aminoethoxy)methyl]-4-(2-chlorophenyl)-3-ethoxycarbonyl-5-methoxycarbonyl-6-methyl-1,4-dihydropyridine and salts thereof, and their use as anti-ischaemic and antihypertensive agents.

U.S. 3,591,584 discloses piroxicam, 4-hydroxy-2-methyl-N-2-pyridinyl-2H-1,2-benzothiazine-3-carboxamide 1,1-dioxide, and its use as an antiinflammatory and analgesic agent.

Pertinent prodrug forms of piroxicam are disclosed in U.S. 4,309,427 and U.S. 4,563,452.

U.S. 4,188,390 discloses doxazosin, 4-amino-2-[4-(1,4-benzodioxan-2-carbonyl)piperazin-1-yl]-6,7-dimethoxyquinazoline, and its use as a regulator of the cardiovascular system, particularly in treatment of hypertension.

Use of glipizide, 1-cyclohexyl-3-[p-[2-(5-methyl-pyrazinecarboxamido)ethyl]-phenylsulfonyl]urea, as an antidiabetic agent is disclosed in U.S. 3,669,966.

The present invention provides novel advantageous transdermal flux enhancing pharmaceutical compositions for transdermal administration to humans or lower animal subjects. The compositions of the invention may incorporate any of a wide variety of pharmacologically active compounds or prodrugs thereof. Thus, the instant compositions comprise a safe and effective amount of a pharmacologically active compound or a prodrug thereof, an aqueous solvent system comprising from about 15 to 75% by volume of one or more water miscible solvents and from about 0.01 to 5% (w/v) of a penetration enhancer selected from a 1-alkylazacycloheptan-2-one wherein said alkyl has from 8 to 16 carbon atoms, and a cis-olefin compound of the formula

$$CH_3(CH_2)_xCH=CH(CH_2)_yR^3$$

where $R^3$ is $CH_2OH$, $CH_2NH_2$ or $COR^4$ and $R^4$ is OH or ($C_1$-$C_4$)alkoxy, x and y are each an integer from 3 to 13 and the sum of x and y is from 10 to 16. Especially surprising features of the invention are that for a given pharmacologically active compound or prodrug there appears to be a certain concentration of solvent(s) within the above range at which the transdermal flux is optimal and that the solvent system employed must be aqueous. Thus, the composition of the invention is one in which the concentration of the solvent or solvents is within plus or minus 10% of the concentration which gives optimum transdermal flux for that particular pharmacologically active compound or prodrug. While the entire range of about 15 to 75% for the concentration of the solvent or combined solvents, ordinarily gives markedly improved transdermal flux in comparison with solvent levels outside that range, the more limited range is a "window" within which transdermal flux is found to be most beneficial.

The aqueous solvent system of the invention comprises water and one or more water miscible solvents.

Such water miscible solvents include, but are not limited to, methanol, ethanol, isopropyl alcohol, propylene glycol, polyethylene glycol and glycerin. When the aqueous solvent system comprises one water miscible solvent such solvent is not ethanol and when it comprises two water miscible solvents such solvents are not ethanol and polyethylene glycol. Preferred solvents for this invention are those that are least damaging to skin and include ethanol and glycerin. The water used in this invention may be buffered and the pH adjusted to optimize stability of the particular pharmacologically active compound or prodrug and to reduce or eliminate damage to skin. If the water is buffered, it is preferred that the water be buffered to about pH 6.5 to pH 7.5. Anionic buffers are preferable for such purpose. An appropriate pharmaceutically acceptable anionic buffer is Sorensen's Buffer which comprises $NaH_2PH_4.H_2O$, $Na_2HPO_4$ and NaCl and which is well known to those skilled in the art. Certain cationic buffers such as Tris also can be employed but it has been found that Tris reduces the effect of oleic acid on stratum corneum lipids in a concentration dependent manner.

The ratio of water to solvent or solvents for optimum flux will vary to some extent as a function of the solvent(s), penetration enhancer and pharmacologically active compound or prodrug of the particular composition. The range of ratios for water/solvent(s) within the scope of this invention is from 15/85 (% v/v) to 85/15 (% v/v).

While the present invention is useful for compositions containing a wide variety of pharmacologically active compounds and prodrugs, it is especially useful for compositions used in treatment of humans or lower animals suffering from rheumatic or inflammatory conditions, ischaemic heart disease, especially angina, hyptertension or diabetes.

Especially useful pharmacologically active compounds or prodrugs for the invention compositions include methyl salicylate, salicylic acid, ibuprofen, piroxicam and prodrugs of piroxicam, and pharmaceutically acceptable cationic and acid addition salts thereof, for treatment of rheumatic or inflammatory conditions. Especially useful prodrugs of piroxicam are those of the formula

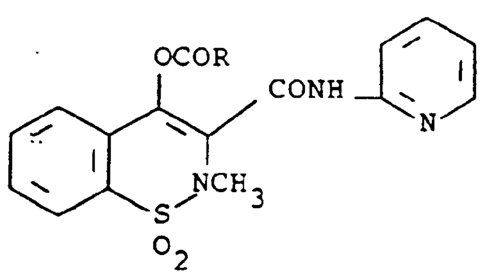
,
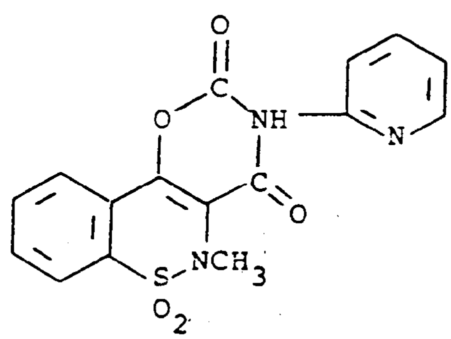

and pharmaceutically acceptable acid addition salts thereof where R is $C_1$ to $C_9$ alkyl, which may be a straight chain or branched alkyl, $CH(R^1)OCOR^2$, where $R^1$ is H or $C_1$ to $C_3$ alkyl and $R^2$ is $C_1$ to $C_4$ alkyl or $C_1$ to $C_4$ alkoxy.

Other preferred compositions of the invention, useful in treating ischaemic heart disease, especially angina, or hypertension in human or lower animals in need of such treatment, are those employing amlodipine, which is disclosed in U.S. 4,572,909.

Further preferred compositions of the invention are those incorporating a safe and effective amount of glipizide for treatment of diabetic conditions. This pharmacologically active compound and its use for treatment of diabetic conditions is known from U.S. 3,669,966. Yet further preferred compositions of the invention are those employing a safe and effective amount of doxazosin, useful in a preferred method of the invention for treatment of hypertension. The compound and its antihypertensive applications are disclosed in U.S. 4,188,390.

Ester prodrugs of piroxicam are disclosed in U.S. 4,309,427. U.S. 4,563,452 discloses the above oxazino[5,6-c]1,2-benzothiazine prodrug forms of piroxicam.

A particularly preferred class of penetration enhancers useful in the invention compositions are the cis-monoenoic acids of the formula

$$CH_3(CH_2)_xCH=CH(CH_2)_yCOOH$$

wherein x and y are as defined above, and the above 1-alkylazacycloheptan-2-ones wherein said alkyl has from 10 to 14 carbon atoms. Especially preferred members within this class of penetration enhancers are cis-9-tetradecenenoic acid, cis-6-pentadecenoic acid, cis-6-hexadecenoic acid, cis-9-hexadecenoic acid, cis-9-octadecenoic acid (oleic acid), cis-6-octadecenoic acid, cis-11-octadecenoic acid, cis-12-octadecenoic acid, cis-5-eicosenoic, cis-9-eicosenoic acid, cis-11-eicosenoic acid, cis-14-eicosenoic acid, 1-decylazacycloheptan-2-one, 1-dodecyclazacycloheptan-2-one and 1-tetradecyclazacycloheptan-2-one.

Most particularly preferred penetration enhancers because of their efficacy and ease of availability are cis-9-octadecenoic acid (oleic acid), cis-11-octadecenoic acid (cis-vaccenic acid), and 1-dodecylazacycloheptan-2-one, also referred to herein as Azone.

A preferred range of concentration of water miscible solvent or combined water miscible solvents for providing optimum transfermal flux of physiologically active compounds and prodrugs thereof in the invention compositions is from 20 to 60% by volume.

A particularly preferred range of concentration for the penetration enhancers of the invention is from 0.1 to 1% w/v and especially from 0.25 to 0.5% w/v for reasons of efficiency and lack of irritation.

The pharmacologically active compound may be methyl salicylate, salicylic acid, ibuproten, amlodipine, glipizide, doxazosin, piroxicam, a prodrug of piroxicam or a pharmaceutically acceptable cationic or acid addition salt thereof.

A safe and effective amount of a pharmacologically active compound or prodrug for use in the pharmaceutical compositions of the invention is understood herein to mean an amount that will provide therapeutically useful blood and/or local levels of the active compound by the transdermal route of administration. The therapeutically useful levels for the individual pharmacologically active compounds and prodrugs are those known in the art to be useful for each of such compounds. Said pharmaceutical compositions can assume a variety of forms, e.g, a solution, gel or suspension of the active compound or prodrug.

A prodrug of a physiologically active compound herein means a structurally related compound or derivative of an active compound which is absorbed into the human or lower animal body where it is converted to the desired physiologically active compound. The prodrug itself may have little or none of the desired activity.

Within the scope of sound medical judgment the amount of a given physiologically active compound or prodrug used will vary with the particular condition being treated, the severity of the condition, the duration of the treatment, the nature of the compound employed, the condition of the patient and other factors within the specific knowledge and expertise of the attending physician.

While the pharmaceutical compositions of the invention can employ a wide variety of physiologically active compounds or prodrugs thereof, useful in treatment of, for example, fungal and bacterial infections, inflammatory conditions, pain, ischaemic heart disease including angina pectoris and hypertension, allergic conditions and diabetes, a preferred group of physiologically active compounds includes methyl salicylate, salicylic acid, ibuprofen, piroxicam and the above described prodrugs of piroxicam, all of which are useful in treating rheumatic or inflammatory conditions; amlodipine for treatment of ischaemic heart disease, especially angina, or hypertension; glipizide for treatment of diabetes and doxazosin for treatment of hypertension.

Dosage forms for the pharmaceutical compositions of the invention may include solutions, lotions, ointments, creams, gels, suppositories, rate-limiting sustained release formulations and devices therefor.

In addition to the requisite solvent(s), water and penetration enhancer for the compositions of the invention, typical dosage forms may include inert carriers such as gel-producing materials, mineral oil, emulsifying agents and benzyl alcohol. Specific illustrations of several such formulations are set forth in the examples, below.

The pharmaceutically acceptable salts of the above mentioned physiologically active compounds include both cationic salts of those compounds containing an acidic group such as a carboxylic acid, and acid addition salts of those compounds containing a basic nitrogen atom.

By pharmaceutically acceptable cationic salts is meant the salts formed by neutralization of the free carboxylic acid group of the pharmacologically active compounds e.g, salicylic acid and ibuprofen. The neutralization is brought about by contacting said carboxylic acid containing compounds with a base of a pharmaceutically acceptable metal, ammonia or amine. Examples of such metals are sodium, potassium, calcium and magnesium. Examples of such amines are N-methylglucamine and ethanolamine.

By the term pharmaceutically acceptable acid addition salts is meant those salts formed between the free amino group of the above physiologically active compounds (e.g piroxicam, amlodipine and doxazosin) and a pharmaceutically acceptable acid. Examples of such acids are acetic, benzoic, hydrobromic, hydrochloric, citric, fumaric, maleic, succinic, tartaric, benzenesulfonic, p-toluenesulfonic and methanesulfonic acids.

The invention is illustrated by Examples 3 to 8 given below. Examples 1 and 2 describe experimental procedures used in the subsequent Examples.

Skin Samples for Penetration Studies

Male, hairless mice, 8 to 16 weeks of age, were sacrificed by cervical dislocation. A section of full-thickness abdominal skin was surgically excised and mounted between two identical diffusion half-cells[1] having 1.0 $cm^2$

[1]Side-by-side cells obtained from Crown Glass Co., Somerville, New Jersey.

surface area. The skins were then hydrated for about 18 hours with Sorensen's isotonic buffer (0.067M sodium phosphate, pH 7.38) prior to conducting experiments. Human skin, taken in surgery or autopsy, was dermatoned to about 400 micrometers (μm) thickness and hydrated in the same manner.

Stratum corneum sheets were prepared from porcine or human skin by trypsin treatment. Thus, full thickness skin samples were dermatomed to a thickness of 350-400 μm, spread, stratum corneum side up, on filter paper saturated with 0.5% crude trypsin[2] in phosphate buffered saline, pH 7.4. After several hours at 37°C., the stratum corneum layer was peeled away from underlying layers, washed in soybean trypsin inhibitor and several changes of distilled water and spread on wire mesh to dry. Samples were stored desiccated at room temperature until used.

## EXAMPLE 1

### Correlation of Effects of Various Fatty Acids on Flux Enhancement of Salicylic acid, Infrared Spectral Data and Differential Scanning Calorimetry with Porcine Stratum Corneum

Stratum corneum sheets were prepared from porcine skin by trypsin treatment. Thus, full thickness porcine skin samples were dermatomed to 350 μm thickness and spread, stratum corneum side up, on filter paper saturated with 0.5% crude trypsin in phosphate buffered saline at pH 7.4 (Sorensen's buffer). After several hours at 37°C. the stratum corneum was peeled away, washed in soybean trypsin inhibitor, water and air dried. Samples were stored desiccated at room temperature until used. Prior to use, dry skin samples of known weight were incubated for two hours in an 0.15M solution of the appropriate fatty acid in ethanol, the samples were then washed for ten seconds in ethanol, spread on wire mesh, dried over a desiccant and the dry sample reweighed. The stratum corneum samples were then held for several days in a chamber at 22°C., 95% relative humidity, during which the stratum corneum samples equilibrated to a water content of 30% (w/w).

### Infrared Spectral Data

Infrared spectra were obtained with a Fourier Transform Infrared Spectrometer[4] (FTIR) equipped with a liquid nitrogen cooled mercury-cadmium telluride detector. In order to prevent water loss, hydrated samples were sealed between zinc sulfide windows while maintained at 22°C., 95% relative humidity. Sealed samples were placed in the spectrometer where an average of 127 scans were obtained in about six minutes for each of the fatty acid treatments. The digitized data were transferred to a computer (Apple IIe) for determination of frequency and bandwidth of the C-H . antisymmetric stretching absorbance. Due to the digital nature of the FTIR instrument, absorbance and frequency data exist only in discrete increments. With the instrument used, the exact value of any frequency point could only be determined with a precision not greater than 2.7 $cm^{-1}$. The peak frequency was estimated with much greater precision, however, using a center of gravity algorithm for digitized data reported by Cameron et al., Applied Spectr., 36 245-250 (1982).

### Differential Scanning Calorimetry (DSC)

The differential scanning calorimeter[5] was used at a scan rate of 0.75°C./minute. Duplicate samples from each of the above FTIR experiments were combined for DSC measurements. Alternately, stratum corneum samples of known weight (about 20 mg) were treated with each fatty acid in the same manner described above. Treated samples were hydrated for several days at 95% R.H., 22°C. and reweighed. Results show approximately 30% (w/w) water uptake regardless of fatty acid employed.

### Flux Method

Sheets of excised porcine skin cut to 350 μm thickness were mounted between two halves of a diffusion cell with the stratum corneum side toward the donor compartment which contained 1.0 ml of saturated salicylic acid in ethanol (0.31 grams/ml) plus about $10^5$dpm[6]/ml of $^{14}$C-labeled salicylic acid. The appropriate fatty acid was then added to give a final concentration of 0.15M. The receiver compartment contained 1.0 ml Sorensen's buffer, pH 7.4. Both compartments were stirred with a magnetic stirrer and maintained at 32°C.

[2]Type II from Sigma Chemical, St. Louis, MO 63178, USA.
[4]Analect model FX-6200, Laser Precision Corp., Irvine, California.
[5]Microcal model MC-1, Microcal Inc., Amherst, Massachusetts.

$$^{6}\text{dpm} = \text{disintegration per minute},$$

$$\text{dpm} = \frac{\text{photons counts per minute}}{\text{efficiency of the counting}}$$

Samples were removed periodically from the receiver side of the diffusion cell, mixed with a scintillation cocktail (Scintisol, Isolabs, Inc., Akron, OH) and counted for several minutes in a liquid scintillation counter (Model Mark III-6881, Tracor Analytical, Elk Grove Village, IL). Following an initial lag time of about 6 hours, the amount of salicylic acid appearing in the receiver side was linear with time for the duration of the experiment (routinely 24 to 48 hours). From a linear least squares analysis of these data the rate of appearance of salicylic acid in the receiver (dpm/h) was determined. This value, when divided by the specific acitivity of salicyclic acid in the saturated solution (approximately 300 dpm/mg) and the area of exposed skin (0.2 $cm^2$), yielded the flux ($mg/cm^2/h$). Samples removed from the donor side at the beginning and end of the experiment contained, within error, the same amount of salicylic acid. Thus, constant concentration of the permeant was maintained on the donor side throughout the experiment.

The results of all three studies are summarized in Table I

TABLE I

A summary of spectral, thermal and flux changes following treatment of porcine stratum corneum with fatty acids of 18 carbon length. The IR and DSC results were obtained with samples hydrated to 30% (w/w) water content. For the monounsaturated acids, the form (cis vs. trans) and position along the carbon chain of each isomer is shown in parentheses. Each value represents the average of at least two samples.

| Treatment | Peak IR Frequency ($cm^{-1}$) | DSC $T_m$ ≠ (°C) | Flux of Salicyclic Acid ($mg/cm^2/h$) |
|---|---|---|---|
| Stearic | *2918.0 ± 0.4 | *62.5 ± 1.0 | 1.21 |
| Petroselenic (cis-6,7) | 2919.0 | 60.5 | 0.79 |
| Petroseladic (trans-6,7) | 2919.0 | 62.0 | 0.97 |
| Oleic (cis-9,10) | *2920.0 ± 0.5 | *59.0 ± 1.5 | 3.81 |
| Elaidic (trans-9,10) | 2919.4 | 61.5 | 2.35 |
| cis-vaccenic (cis-11,12) | 2920.1 | 57.0 | 5.53 |
| trans-vaccenic (trans-11,12) | 2818.8 | 61.0 | 1.11 |
| Ethanol | *2918.8 ± 0.4 | *62.0 ± 1.0 | 1.31 |
| No Treatment | 2918.8 | 62.0 | --- |

* -Value represents the average ± SEM of three samples.

≠ Temperature of the transition maximum.

Oleic and cis-vaccenic acids each gave a maximum infrared absorbance at 2920 $cm^{-1}$ while the saturated stearic acid and the two trans-acids cave lower values (about 2918-2919), as did the controls. While the differences between the groups of fatty acids is less than the digital resolution of the instrument (2.7 $cm^{-1}$), the center of gravity technique of peak frequency determination allows sufficient precision to easily estimate differences of less than 1.0 $cm^{-1}$ from digitized data. Furthermore, several of the experiments were repeated in triplicate with a standard error of the mean of less than 0.5 $cm^{-1}$. Thus, while small, the peak frequency changes following treatment of stratum corneum with oleic and cis-vaccenic acid compared to the others, are significant.

From the DSC data it is also seen that the two cis-fatty acids show decreased temperature of the transition maxima when compared to stearic acid, the two trans-fatty acids and the controls. It was also noted that the cis-fatty acids gave a broader peak (ratio of peak width to peak height) than did others. The data also suggests that increasing the distance of the double bond from the carboxyl group gives rise to a larger decrease in Tm.

The flux data for cis-9-octadecenoic acid (oleic acid) is also significantly greater than that of stearic acid, the ethanol control and elaidic acid. The difference in flux rates is even greater for cis-vaccenic acid relative to the controls and trans-vaccenic acid. Thus, the above infrared and DSC results each show a high degree of correlation with flux rate.

EXAMPLE 2

Correlation of Lipid Melting Temperature by DSC with Ethanol Concentration of Aqueous Vehicles Containing Oleic Acid

Employing the above procedure for obtaining lipid transition temperature of porcine stratum corneum samples by differential scanning calorimetry, the melting temperature, Tm, for stratum corneum in various ethanol/Sorensen's buffer solutions, each containing 0.25% v/v cis-9-octadecenoic acid (oleic acid) (0.22 w/v), were obtained. The results are summarized in the following table.

| % Ethanol (v/v) in Ethanol/Buffer* Vehicles Containing 0.25% v/v Oleic Acid | Porcine Stratum Corneum Lipid Transition Temperature, Tm, °C. |
|---|---|
| 0/100 | 57.5 |
| 20/80 | 54.5 |
| 30/70 | 54.0 |
| 40/60 | 53.2 ± 0.6 |
| 50/50 | 55.1 |
| 60/40 | 53.4 |
| 70/30 | 58.8 |
| 100/0 | 66.4 |

*Sorensen's Buffer, pH 7.3.

Under the same conditions, stratum corneum samples in Sorensen's buffer alone (no ethanol or cis-9-octadecenoic acid (oleic acid)) gave a Tm of 64°C. Stratum corneum in a vehicle containing 40/60 v/v ethanol/buffer with no cis-9-octadecenoic acid (oleic acid) also had a Tm of 64°C.

The above results, strongly suggest that the 20-70% v/v ethanol vehicles, and especially those having 30-60% ethanol, have a unique ability to disrupt the stratum corneum, a property which is indicative of enhancement of transdermal flux.

EXAMPLE 3

Solution formulations are prepared as follows:

A. Oleic acid 0.25 g
Amlodipine benzenesulfonate 1.0 g
Propylene glycol 40.0 ml
Water q.s. to 100 ml
NaOH q.s. to adjust to pH 5.0

B. Oleic acid 0.25 g
Piroxicam 1.0 g
Glycerin 40.0 ml
Water q.s. to 100 ml
NaOH q.s. to adjust to pH 7.5

C. Oleic acid 0.25 g
Piroxicam 1.0 g
Ethanol 20.0 ml
Propylene glycol 40.0 ml
Water q.s. to 100 ml
NaOH q.s. to adjust to pH 7.5

D. Oleic acid 0.5 g

Piroxicam 1.0 g
Ethanol 20.0 ml
Glycerin 40.0 ml
Water q.s. to 100 ml
NaOH q.s. to adjust to pH 7.5

E. Oleic acid 0.25 g
Piroxicam 1.0 g
Ethanol 20.0 ml
Propylene glycol 40.0 ml
Phosphoric acid 0.1 ml
Water q.s. to 100 ml
NaOH q.s. to adjust to pH 7.5

EXAMPLE 4

The following are illustrative formulations for gels of the invention compositions.

A. Oleic acid 0.25 g or Azone 0.50 g
Carbopol 940[8] 0.7 g
Benzyl alcohol 1.0 g
Diisopropanolamine 1.1 g
Hydroxyethylcellulose 0.4 g
piroxicam 1.0 g
Ethanol 30.0 ml
Water q.s. to make 100 ml

The ingredients are combined, warmed while stirring to effect dispersion and allowed to cool to room temperature.

B. Oleic acid 0.25 g or Azone 0.50 g
Carbopol 940 0.7 g
Benzyl alcohol 1.0 g
Diisopropanolamine 1.1 g
Hydroxyethylcellulose 0.4 g
Amlodipine benzenesulfonate 1.0 g
Ethanol 35 ml
Water q.s. to make 100 ml

The ingredients were treated as in A, above to form the desired gel.

When 0.8 g of glipizide or 1.0 g ibuprofen, 3.0 g salicylic acid 0.9 g of doxazosin mesylate are used in place of amlodipine benzenesulfonate in the above formulation satisfactory gels are obtained in like manner.

C. Penetration enhancer[9] 0.01 to 5.0 g
Carbopol 940 1.0 g
Benzyl alcohol 1.0 g
Diisopropanolamine 1.0 g
Hydroxyethylcellulose 0.5 g
One or more water miscible solvents[10] 15 to 75 ml
Methyl salicylate 10 g
Water q.s. to make 100 ml

D. Oleic acid 0.25 g
Carbopol 940 0.70 g
Benzyl alcohol 1.0 g

[8]Carbopol 940 is a polyacrylic acid polymer available from B.F. Goodrich Co., Inc.

[9]Penetration enhancers include cis-9-octadecenoic acid (oleic acid), cis-6-octadecenoic, cis-11-octadecenoic, cis-12-octa-decenoic, cis-5-eicosenoic, cis-9-eicosenoic, cis-11-eicosenoic and cis-14-eicosenoic acids; 1-decylazacycloheptan-2-one, 1-dodecylazacycloheptan-2-one and 1-tetradecylazacycloheptan-2-one, cis-9-octadecenylamine, cis-11-octadecenylamine, cis-14-eicosenylamine, cis-9-tetradecenyl alcohol, cis-11-octadecenyl alcohol, ethyl oleate, ethyl cis-5-eicosenoate, methyl cis-12-octadecenoate, isopropyl cis-9-hexadecenoate and n-butyl cis-9-tetradecenoate.

[10]Water miscible solvents include methanol, ethanol isopropyl alcohol, propylene glycol, polyethylene glycol and glycerin.

Diisopropanolamine 1.0 g
Hydroxyethylcellulose 0.4 g
Piroxicam 0.5 g
Ethanol 25.0 ml
Propylene glycol 20.0 ml
Water q.s. to 100 ml

The ingredients are treated as in A, above, to form the desired gel.

E. Oleic acid 0.25 g
Carbopol 934 0.70 g
Benzylalcohol 1.0 g
Triethanolamine 1.1 g
Hydroxyethylcellulose 0.4 g
Piroxicam 1.0 g
Glycerin 30.0 ml
Water q.s. to 100 ml

The ingredients are treated as in A, above, to form the desired gel.

EXAMPLE 5

The following formulations are illustrative of hydrophilic ointments as dosage forms of the compositions of the invention.

A. Oleic acid 0.25 g
active ingredient[12] 1-5g
PEG 4000 17.0 g
PEG 400 17.0 g
One or more water miscible solvents[13] 15-55ml
Water q.s. to make 100 ml

B. Oleic acid 0.25 g
Piroxicam 1.0 g
PEG 4000 17.2 g
PEG 200[1]17.2 g
Propylene glycol 30.0 ml
Water q.s. to make 100 ml

EXAMPLE 6

Correlation of Lipid Melting Temperature by DSC with Glycerin Concentration of Aqueous Vehicles Containing Oleic Acid

Employing the procedure of Example 1, above, for obtaining lipid transition temperature of porcine stratum corneum samples by differential scanning calorimetry, the melting temperature, Tm, for stratum corneum in various glycerin/0.1M Tris buffer solutions (Ph 6.8-7.3) each containing 0.25% v/v cis-9-octadecenoic acid (oleic acid) (0.22 w/v), were obtained. The results are summarized in the following table.

[12]Active ingredients include methyl salicylate, salicylic acid, ibuprofen, piroxicam, amlodipine benzenesulfonate, doxazosin mesylate and glipizide.

[13]Water miscible solvents include methanol, isopropyl alcohol, propylene glycol, polyethylene glycol and glycerin.

PEG 200 is commercial polyethylene glycol of molecular weight 190-210. PEG 400 is commercial polyethylene glycol of molecular weight 380-420. PEG 4000 is commercial polyethylene glycol, M.W. 3000-3700.

| % Glycerin (v/v) in 0.1M Tris Buffer (pH 6.8-7.3) Vehicles Containing 0.25% v/v Oleic Acid | Porcine Stratum Corneum Lipid Transition Temperature, Tm, °C |
|---|---|
| 0/100 | 58 |
| 20/80 | 62.5 |
| 40/60 | 57 |
| 60/40 | 54 |
| 80/20 | 59 |

Under the same conditions, stratum corneum samples in 0.1M Tris buffer (pH 6.8-7.3) alone (no glycerin or cis-9-octadecenoic acid (oleic acid)) gave a Tm of 61°C.

The above results demonstrate that those vehicles having about 40-60% glycerin have the ability to disrupt the stratum corneum, a property which, as discussed in Example 2, is indicative of enhancement of transdermal flux.

EXAMPLE 7

Correlation of Lipid Melting Temperature by DSC with Polyethylene Glycol 200 (PEG 200) Concentration of Aqueous Vehicles Containing Oleic acid

Employing the procedure of Example 1, above, for obtaining lipid transition temperature of porcine stratum corneum samples by differential scanning calorimetry, the melting temperature, Tm, for stratum corneum in various PEG 200/0.1M Tris buffer solutions (pH 6.8-7.3) each containing 0.25% v/v cis-9-octadecenoic acid (oleic acid) (0.22 w/v) were obtained. The results are summarized in the following table.

| % PEG 200 (v/v) in 0.1M Tris Buffer (pH 6.8-7.3) Vehicles Containing 0.25% v/v Oleic Acid | Porcine Stratum Corneum Lipid Transition Temperature, Tm, °C |
|---|---|
| 0/100 | 59 |
| 20/80 | 59 |
| 40/60 | 57.5 |
| 60/40 | 57 |
| 80/20 | 61 |

Under the same conditions, stratum corneum samples in 0.1M Tris buffer (pH 6.8-7.3) alone (no PEG 200 or cis-9-octadecenoic acid (oleic acid)) gave a Tm of 61°C.

The above results demonstrate that those vehicles having about 40-60% PEG 200 have the ability to disrupt the stratum corneum, a property which, as discussed in Example 2, is indicative of enhancement of transdermal flux.

EXAMPLE 8

Correlation of Lipid Melting Temperature by DSC with Ethanol and Propylene Glycol (PG) Concentrations of Aqueous Vehicles Containing Oleic Acid

Employing the procedure of Example 1, above, for obtaining lipid transition temperature of porcine stratum

corneum samples by differential scanning calorimetry, the melting temperature, Tm, for stratum corneum in various ethanol/PG/0.1M Tris buffer solutions (pH 6.8-7.3) each containing 0.25% v/v cis-9-octadecenoic acid (oleic acid) (0.22 w/v) were obtained. The results are summarized in the following table.

| % Ethanol and PG (v/v/v) in 0.1M Tris Buffer (pH 6.8-7.3) Vehicles Containing 0.25% v/v Oleic Acid | Porcine Stratum Corneum Lipid Transition Temperature, Tm, °C |
|---|---|
| 40/20/40 | 58.5 |
| 33/33/34 | 59 |
| 66/34/0 | 60 |
| 20/40/40 | 55 |
| 40/40/20 | 60 |
| 34/66/0 | 61 |

Under the same conditions, stratum corneum samples in 0.1M Tris buffer (pH 6.8-7.3) alone (no ethanol or PG or cis-9-octadecenoic acid (oleic acid)) gave a Tm of 62.5°C.

The above results demonstrate aqueous vehicles having two miscible solvents have the ability to disrupt the stratum corneum and that the degree of disruption can vary with the ratio of the solvents to each other (compare 40/20/40 with a Tm of 58.5°C to 20/40/40 with a Tm of 55°C).

## Claims

### Claims for the following Contracting States : AT, BE, CH, DE, FR, GB, IT, LI, LU, NL, SE

1. A transdermal flux enhancing pharmaceutical composition for transdermal administration to a human or lower animal subject comprising
   (a) a safe and effective amount of a pharmacologically active compound or a prodrug thereof,
   (b) an aqueous solvent system comprising from about 15 to 75% by volume of one or more water-miscible solvents, and
   (c) from about 0.01 to 5% (w/v) of a penetration enhancer selected from a 1-alkylazacycloheptan-2-one, said alkyl having from 8 to 16 carbon atoms, and a cis-olefin compound of the formula
   $$CH_3(CH_2)_xCH{=}CH(CH_2)_yR^3$$
   where $R^3$ is $CH_2OH$, $CH_2NH_2$ or $COR^4$, and $R^4$ is OH or $(C_1\text{-}C_4)$alkoxy, x and y are each an integer from 3 to 13 and the sum of x and y is from 10 to 16;
   wherein in (b) the solvent or combined solvents content is within plus or minus 10% of that which gives optimum transdermal flux for said compound or prodrug with the proviso that when the aqueous solvent system comprises one water miscible solvent, such solvent is not ethanol and when it comprises two water miscible solvents, such solvents are not ethanol and polyethylene glycol, and when the drug is hydrocortisone or triamcinolone the solvent system does not comprise 25% by weight of propylene glycol and 30% by weight of ethanol.

2. A composition according to claim 1, wherein said pharmacologically active compound is methyl salicylate, salicylic acid, ibuproten, amlodipine, glipizide, doxazosin, piroxicam, a prodrug of piroxicam or a pharmaceutically acceptable cationic or acid addition salt thereof.

3. The composition according to claim 1 or 2 wherein in (b) the aqueous solvent system comprises from 20 to 60% by volume of one or more water miscible solvents.

4. A transdermal flux enhancing pharmaceutical composition according to claim 2 wherein said prodrug is a prodrug of piroxicam of formula:

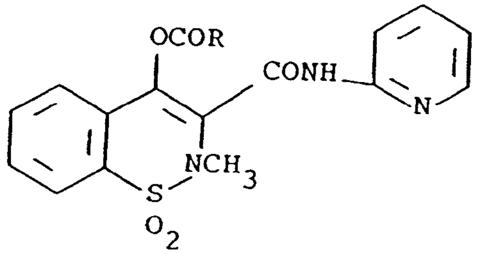

or

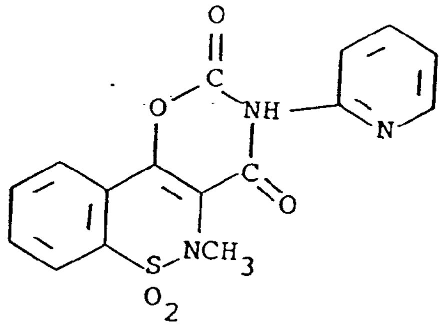

or a pharmaceutically acceptable cationic and acid addition salt thereof;

where R is $C_1$ to $C_9$ straight chain or branched alkyl or $CH(R^1)OCOR^2$ where $R^1$ is H or ($C_1$-$C_3$)alkyl, and $R^2$ is $C_1$-$C_4$ alkyl or $C_1$-$C_4$ alkoxy.

**5.** A composition according to claim 2 wherein said penetration enhancer is oleic acid, cis-11-octadecenoic acid or 1-dodecylazacycloheptan-2-one.

**Claims for the following Contracting States : ES, GR**

**1.** A process for preparing a transdermal flux enhancing pharmaceutical composition for transdermal administration to a human or lower animal subject which comprises mixing

(a) a safe and effective amount of a pharmacologically active compound or a prodrug thereof,

(b) an aqueous solvent system comprising from about 15 to 75% by volume of one or more water-miscible solvents, and

(c) from about 0.01 to 5% (w/v) of a penetration enhancer selected from a 1-alkylazacycloheptan-2-one, said alkyl having from 8 to 16 carbon atoms, and a cis-olefin compound of the formula

$$CH_3(CH_2)_xCH{=}CH(CH_2)_yR^3$$

where $R^3$ is $CH_2OH$, $CH_2NH_2$ or $COR^4$, and $R^4$ is OH or ($C_1$-$C_4$)alkoxy, x and y are each an integer from 3 to 13 and the sum of x and y is from 10 to 16;

wherein in (b) the solvent or combined solvents content is within plus or minus 10% of that which gives optimum transdermal flux for said compound or prodrug with the proviso that when the aqueous solvent system comprises one water miscible such solvent is not ethanol and when it comprises two water miscible solvents such solvents are not ethanol and polyethyleneglycol, and when the drug is hydrocortisone or triamcinolone the solvent system does not comprise 25% by weight of propylene glycol and 30% of weight by ethanol.

**2.** A process according to claim 1, wherein a composition according to claim 1 is prepared, wherein said pharmacologically active compound is methyl salicylate, salicylic acid, ibuproten, amlodipine, glipizide, doxazosin, piroxicam, a prodrug of piroxicam or a pharmaceutically acceptable cationic or acid addition salt thereof.

**3.** The process according to claim 1 wherein in (b) the aqueous solvent system comprises from 20 to 60% by volume of one or more water miscible solvents.

**4.** A process according to claim 2 wherein a transdermal flux enhancing pharmaceutical composition according to claim 2 wherein said prodrug is a prodrug of piroxicam of formula:

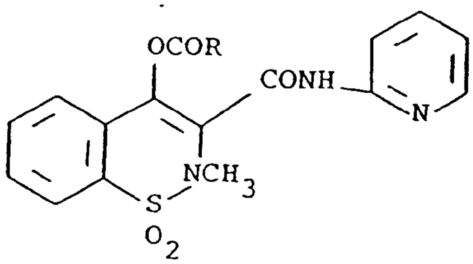

or

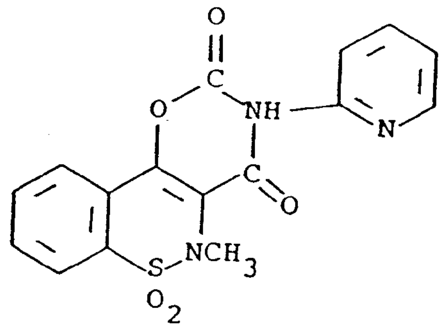

or a pharmaceutically acceptable cationic and acid addition salt thereof;

where R is $C_1$ to $C_9$ straight chain or branched alkyl or $CH(R^1)OCOR^2$ where $R^1$ is H or $(C_1$-$C_3)$alkyl, and $R^2$ is $C_1$-$C_4$ alkyl or $C_1$-$C_4$ alkoxy.

5. A process according to claim 2 wherein said penetration enhancer is oleic acid, cis-11-octadecenoic acid or 1-dodecylazacycloheptan-2-one.

## Patentansprüche

### Patentansprüche für folgende Vertragsstaaten : AT, BE, CH, DE, FR, GB, IT, LI, LU, NL, SE

1. Eine den transdermalen Penetrationsfluß erhöhende pharmazeutische Zusammensetzung für transdermale Verabreichung an einen menschlichen Patienten oder an ein niederes Tier, enthaltend
   (a) eine sichere und wirksame Menge einer pharmakologisch aktiven Verbindung oder einer Vorstufe derselben,
   (b) ein wässeriges Lösungsmittelsystem, enthaltend von etwa 15 bis 75 Volumprozent eines oder mehrerer mit Wasser mischbarer Lösungsmittel, und
   (c) von etwa 0,01 bis 5 % (Gew./Vol.) eines Penetrationserhöhers, ausgewählt aus einem 1-Alkylazacycloheptan-2-on, worin das Alkyl von 8 bis 16 Kohlenstoffatome enthält, und einer cis-Olefin-Verbindung der Formel

$$CH_3(CH_2)_xCH{=}CH(CH_2)_yR^3$$

   worin $R^3$ die Bedeutung von $CH_2OH$, $CH_2NH_2$ oder $COR^4$ hat und $R^4$ eine $(C_1$-$C_4)$-Alkoxygruppe oder OH ist, jeder der Indizes x und y eine ganze Zahl mit einem Wert von 3 bis 13 bedeutet und die Summe von x und y einen Wert von 10 bis 16 aufweist;

   worin in (b) das Lösungsmittel oder der kombinierte Gehalt an Lösungsmitteln innerhalb von plus oder minus 10 % von dem Gehalt liegt, welcher einen optimalen transdermalen Fluß für die erwähnte Verbindung oder deren Vorstufe liefert, mit der Maßgabe, daß, wenn das wässerige Lösungsmittelsystem ein mit Wasser mischbares Lösungsmittel enthält, ein solches Lösungsmittel nicht Ethanol ist, und wenn es zwei mit Wasser mischbare Lösungsmittel enthält, derartige Lösungsmittel nicht Ethanol und Polyethylenglykol sind, und sofern das Arzneimittel Hydrocortison oder Triamcinolon ist, das Lösungsmittelsystem nicht 25 Gewichtsprozent Propylenglykol und 30 Gewichtsprozent Ethanol enthält.

2. Zusammensetzung nach Anspruch 1, worin die pharmakologisch aktive Verbindung Methylsalicylat, Salicylsäure, Ibuproten, Amlodipin, Glipizid, Doxazosin, Piroxicam, eine Vorstufe von Piroxicam oder ein pharmazeutisch verträgliches kationisches oder Säureadditionssalz davon ist.

3. Zusammensetzung nach Anspruch 1 oder 2, worin in (b) das wässerige Lösungsmittelsystem von 20 bis 60 Volumprozent von einem oder mehreren mit Wasser mischbaren Lösungsmitteln enthält.

4. Eine den transdermalen Penetrationsfluß erhöhende pharmazeutische Zusammensetzung nach Anspruch 2, worin die erwähnte Vorstufe eine Vorstufe von Piroxicam der Formel

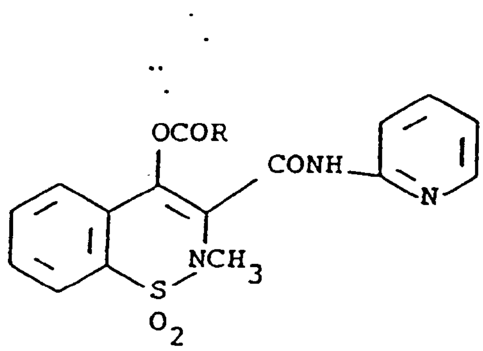

oder

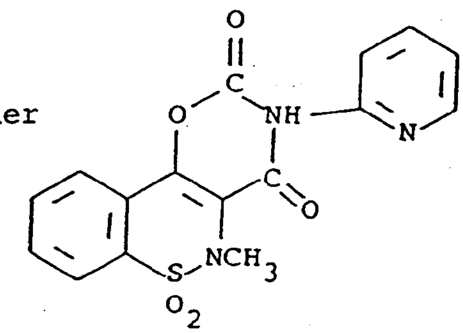

oder ein pharmazeutisch verträgliches kationisches und Säureadditionssalz davon ist;

worin R $C_1$- bis $C_9$-geradkettiges oder verzweigtkettiges Alkyl oder $CH(R^1)OCOR^2$ ist, worin $R^1$ ein ($C_1$-$C_3$)-Alkyl oder H ist und $R^2$ $C_1$-$C_4$-Alkyl oder $C_1$-$C_4$-Alkoxy bedeutet.

**5.** Zusammensetzung nach Anspruch 2, worin der erwähnte Penetrationserhöher Ölsäure, cis-11-Octadecensäure oder 1-Dodecylazacycloheptan-2-on ist.

**Patentansprüche für folgende Vertragsstaaten : ES, GR**

**1.** Ein Verfahren zur Herstellung einer, den transdermalen Penetrationsfluß erhöhenden pharmazeutischen Zusammensetzung für transdermale Verabreichung an einen menschlichen Patienten oder an ein niederes Tier, welches umfaßt das Mischen

(a) einer sicheren und wirksamen Menge einer pharmakologisch aktiven Verbindung oder einer Vorstufe derselben,

(b) eines wässerigen Lösungsmittelsystems, enthaltend von etwa 15 bis 75 Volumprozent eines oder mehrerer mit Wasser mischbarer Lösungsmittel, und

(c) von etwa 0,01 bis 5 % (Gew./Vol.) eines Penetrationserhöhers, ausgewählt aus einem 1-Alkylazacycloheptan-2-on, worin das Alkyl von 8 bis 16 Kohlenstoffatome enthält, und einer cis-Olefin-Verbindung der Formel

$$CH_3(CH_2)_xCH=CH(CH_2)_yR^3$$

worin $R^3$ die Bedeutung von $CH_2OH$, $CH_2NH_2$ oder $COR^4$ hat und $R^4$ eine ($C_1$-$C_4$)-Alkoxygruppe oder OH ist, jeder der Indizes x und y eine ganze Zahl mit einem Wert von 3 bis 13 bedeutet und die Summe von x und y einen Wert von 10 bis 16 aufweist;

worin in (b) das Lösungsmittel oder der kombinierte Gehalt an Lösungsmitteln innerhalb von plus oder minus 10 % von dem Gehalt liegt, welcher einen optimalen transdermalen Fluß für die erwähnte Verbindung oder deren Vorstufe liefert, mit der Maßgabe, daß, wenn das wässerige Lösungsmittelsystem ein mit Wasser mischbares Lösungsmittel enthält, ein solches Lösungsmittel nicht Ethanol ist, und wenn es zwei mit Wasser mischbare Lösungsmittel enthält, derartige Lösungsmittel nicht Ethanol und Polyethylenglykol sind, und sofern das Arzneimittel Hydrocortison oder Triamcinolon ist, das Lösungsmittelsystem nicht 25 Gewichtsprozent Propylenglykol und 30 Gewichtsprozent Ethanol enthält.

**2.** Verfahren nach Anspruch 1, in welchem eine Zusammensetzung gemäß Anspruch 1 verwendet wird, worin die pharmakologisch aktive Verbindung Methylsalicylat, Salicylsäure, Ibuproten, Amlodipin, Glipizid, Doxazosin, Piroxicam, eine Vorstufe von Piroxicam oder ein pharmazeutisch verträgliches kationisches oder Säureadditionssalz davon ist.

**3.** Verfahren nach Anspruch 1 oder 2, worin in (b) das wässerige Lösungsmittelsystem von 20 bis 60 Volumprozent von einem oder mehreren mit Wasser mischbaren Lösungsmitteln enthält.

**4.** Verfahren nach Anspruch 2, worin eine den transdermalen Penetrationsfluß erhöhende pharmazeutische Zusammensetzung nach Anspruch 2, worin die erwähnte Vorstufe eine Vorstufe von Piroxicam der Formel

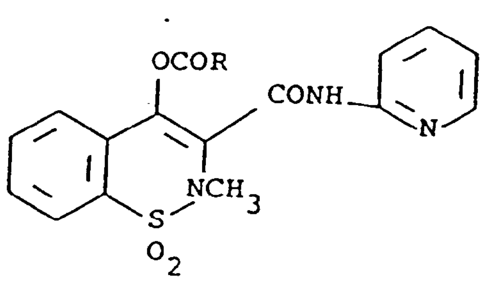

oder

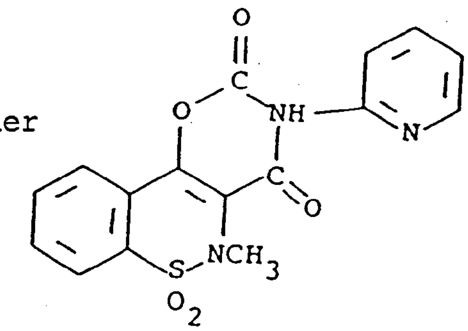

oder ein pharmazeutisch verträgliches kationisches und Säureadditionssalz davon ist;

worin R $C_1$- bis $C_9$-geradkettiges oder verzweigtkettiges Alkyl oder $CH(R^1)OCOR^2$ ist, worin $R^1$ ein ($C_1$-$C_3$)-Alkyl oder H ist und $R^2$ $C_1$-$C_4$-Alkyl oder $C_1$-$C_4$-Alkoxy bedeutet.

5. Verfahren nach Anspruch 2, worin der erwähnte Penetrationserhöher Ölsäure, cis-11-Octadecensäure oder 1-Dodecylazacycloheptan-2-on ist.

## Revendications

### Revendications pour les Etats contractants suivants : AT, BE, CH, DE, FR, GB, IT, LI, LU, NL, SE

1. Composition pharmaceutique destinée à accroître le flux transdermique, pour l'administration transdermique à un sujet humain ou un animal inférieur, comprenant

(a) une quantité sûre et efficace d'un composé pharmacologiquement actif ou d'un précurseur médicamenteux de ce composé,

(b) un solvant aqueux comprenant environ 15 à 75 % en volume d'un ou plusieurs solvants miscibles à l'eau, et

(c) environ 0,01 à 5 % (en poids/volume) d'un agent augmentant la pénétration, choisi entre une 1-alkylazacycloheptane-2-one, dont le groupe alkyle possède 8 à 16 atomes de carbone, et un composé cis-oléfinique de formule

$$CH_3(CH_2)_xCH=CH(CH_2)_yR^3$$

dans laquelle $R^3$ représente un groupe $CH_2OH$, $CH_2NH_2$ ou $COR^4$, et $R^4$ représente un groupe OH ou alkoxy en $C_1$ à $C_4$, x et y représentent chacun un nombre entier de 3 à 13 et la somme de x et y est égale à une valeur de 10 à 16 ;

dans laquelle, en (b), la quantité de solvant ou de mélange de solvants est égale à celle, plus ou moins 10 %, qui provoque le flux transdermique optimal dudit composé ou précurseur médicamenteux sous réserve que : lorsque le solvant aqueux comprend un solvant miscible à l'eau, ce solvant ne soit pas l'éthanol ; lorsqu'il comprend deux solvants miscibles à l'eau, ces solvants ne consistent pas en éthanol et en polyéthylèneglycol ; et lorsque le médicament est l'hydrocortisone ou la triamcinolone, le solvant ne comprenne pas 25 % en poids de propylèneglycol et 30 % en poids d'éthanol.

2. Composition suivant la revendication 1, dans laquelle le composé pharmacologiquement actif est le salicylate de méthyle, l'acide salicylique, l'ibuprofène, l'amlodipine, le glipizide, la doxazosine, le piroxicam, un précurseur médicamenteux du piroxicam ou un de ses sels cationiques ou d'addition d'acides pharmaceutiquement acceptables.

3. Composition suivant la revendication 1 ou 2, dans laquelle, en (b), le solvant aqueux comprend 20 à 60 % en volume d'un ou plusieurs solvants miscibles à l'eau.

4. Composition pharmaceutique destinée à accroître le flux transdermique suivant la revendication 2, dans laquelle le précurseur médicamenteux est le précurseur médicamenteux de piroxicam répondant à la formule :

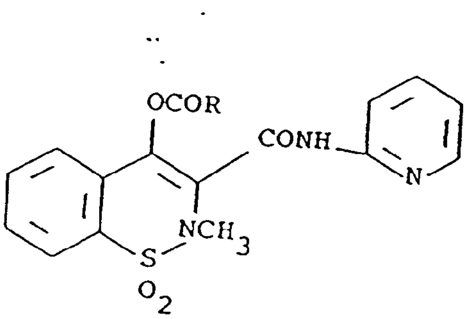 ou 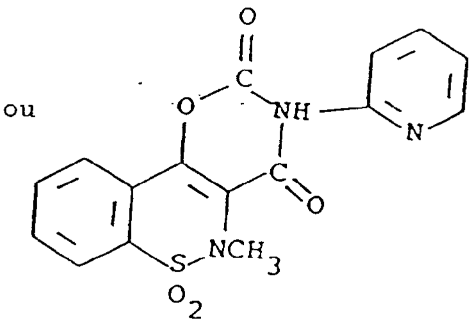

ou un de ses sels cationiques ou d'addition d'acides pharmaceutiquement acceptables ;

formule dans laquelle R représente un groupe alkyle à chaîne droite ou ramifié en $C_1$ à $C_9$ ou un groupe $CH(R^1)OCOR^2$, dans lequel $R^2$ représente H ou un groupe alkyle en $C_1$ à $C_3$, et $R^2$ représente un groupe alkyle en $C_1$ à $C_4$ ou alkoxy en $C_1$ à $C_4$.

**5.** Composition suivant la revendication 2, dans laquelle l'agent augmentant la pénétration est l'acide oléique, l'acide cis-11-octadécénoïque ou la 1-dodécylazacycloheptane-2-one.

**Revendications pour les Etats contractants suivants : ES, GR**

**1.** Procédé de préparation d'une composition pharmaceutique destinée à accroître le flux transdermique, pour l'administration transdermique à un sujet humain ou un animal inférieur, qui consiste à mélanger

(a) une quantité sûre et efficace d'un composé pharmacologiquement actif ou d'un précurseur médicamenteux de ce composé,

(b) un solvant aqueux comprenant environ 15 à 75 % en volume d'un ou plusieurs solvants miscibles à l'eau, et

(c) environ 0,01 à 5 % (en poids/volume) d'un agent augmentant la pénétration, choisi entre une 1-alkylazacycloheptane-2-one, dont le groupe alkyle possède 8 à 16 atomes de carbone, et un composé cis-oléfinique de formule

$$CH_3(CH_2)_xCH{=}CH(CH_2)_yR^3$$

dans laquelle $R^3$ représente un groupe $CH_2OH$, $CH_2NH_2$ ou $COR^4$, et $R^4$ représente un groupe OH ou alkoxy en $C_1$ à $C_4$, x et y représentent chacun un nombre entier de 3 à 13 et la somme de x et y est égale à une valeur de 10 à 16 ;

dans lequel, en (b), la quantité de solvant ou de mélange de solvants est égale à celle, plus ou moins 10 %, qui engendre le flux transdermique optimal dudit composé ou précurseur médicamenteux sous réserve que ; lorsque le solvant aqueux comprend un solvant miscible à l'eau, ce solvant ne soit pas l'éthanol ; lorsqu'il comprend deux solvants miscibles à l'eau, ces solvants ne consistent pas en éthanol et en polyéthylèneglycol ; et lorsque le médicament est l'hydrocortisone ou la triamcinolone, le solvant ne comprenne pas 25 % en poids de propylèneglycol et 30 % en poids d'éthanol.

**2.** Procédé suivant la revendication 1, dans lequel est préparée une composition suivant la revendication 1, dans laquelle le composé pharmacologiquement actif est le salicylate de méthyle, l'acide salicylique, l'ibuprofène, l'amlodipine, le glipizide, la doxazosine, le piroxicam, un précurseur médicamenteux de piroxicam ou un de ses sels cationiques ou d'addition d'acides pharmaceutiquement acceptables.

**3.** Procédé suivant la revendication 1, dans lequel, en (b), le solvant aqueux comprend 20 à 60 % en volume d'un ou plusieurs solvants miscibles à l'eau.

**4.** Procédé suivant la revendication 2, dans lequel est préparée une composition pharmaceutique destinée à accroître le flux transdermique suivant la revendication 2, dans lequel le précurseur médicamenteux est un précurseur médicamenteux de piroxicam répondant à la formule :

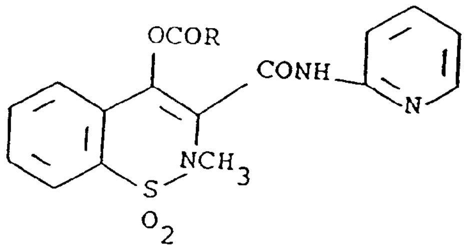

ou

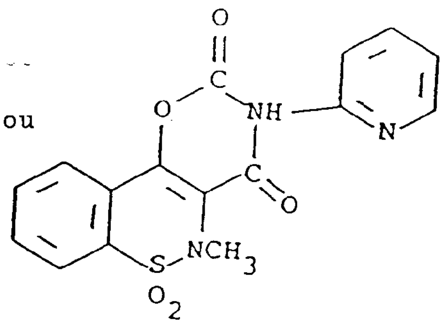

ou un de ses sels cationiques ou d'addition d'acides pharmaceutiquement acceptables ;

formule dans laquelle R représente un groupe alkyle à chaîne droite ou ramifié en $C_1$ à $C_9$ ou un groupe $CH(R^1)OCOR^2$, dans lequel $R^2$ représente H ou un groupe alkyle en $C_1$ à $C_3$, et $R^2$ représente un groupe alkyle en $C_1$ à $C_4$ ou alkoxy en $C_1$ à $C_4$.

**5.** Procédé suivant la revendication 2, dans lequel l'agent augmentant la pénétration est l'acide oléique, l'acide cis-11-octadécènoïque ou la 1-dodécylazacycloheptane-2-one.